# EUROPEAN PATENT APPLICATION

(11) **EP 3 547 804 A1**
(43) Date of publication of application: **02.10.2019**
(21) Application number: 17874917.2
(22) Date of filing: 03.03.2017
(51) Int. Cl.: H05F 3/04

(54) **ION GENERATION DEVICE**

(30) Priority: 28.11.2016 JP 2016230629
(71) Applicant: Sharp Kabushiki Kaisha, Sakai City, Osaka 590-8522 (JP)
(72) Inventor: DATE, Kazuharu, Sakai City, Osaka 590-8522 (JP); HORIKAWA, Kouji, Sakai City, Osaka 590-8522 (JP); TAKADO, Tomoaki, Sakai City, Osaka 590-8522 (JP)
(74) Representative: Treeby, Philip David William
(86) International application number: PCT/JP2017/008547
(87) International publication number: WO 2018/096698

(57) **Abstract**

An embodiment of the present invention provides an ion generating device which can suppress reduction in amount of ions generated and of which size reduction is possible. The ion generating device includes: a discharge electrode (1) which generates positive ions; a discharge electrode (2) which generates negative ions; a transformer (13) which outputs a high alternating-current voltage; a diode (D1) which rectifies the high alternating-current voltage and applies a voltage thus rectified to the discharge electrode (1); and a diode (D2) which rectifies the high alternating-current voltage and applies a voltage thus rectified to the discharge electrode (2). The diode (D1) and the diode (D2) are connected, on a high voltage circuit substrate (14), to an output terminal of the transformer (13) via a conductive body such as a wiring pattern (14a) and a soldering pattern (14b). The conductive body is formed in a region where the conductive body causes neither a decrease in strength of an electric field formed by the discharge electrode (1) nor a decrease in strength of an electric field formed by the discharge electrode (2).

## Description

### Technical Field

The present invention relates to an ion generating device.

### Background Art

An ion generating device is mounted on various electric apparatuses, for various effects of ions generated by the ion generating device. For example, Patent Literature 1 discloses an air purifier which includes an ion generating device.

Further, in recent years, functions of electric apparatuses have been enhanced. Accordingly, an electric apparatus into which electronic control by a microcomputer or the like is introduced has become popular. For example, Patent Literature 2 discloses a dryer which has a build-in control section including a Central Processing Unit (CPU). The control section controls operations of a heater, an ion generating device, etc. Since highly-functional electric apparatuses contain a direct-current component part such as a microcomputer, there are some electric apparatuses whose power source is specified as a direct-current power source. Then, an ion generating device mounted on such an electric apparatus accordingly becomes an ion generating device whose power source is specified as a direct-current power source.

In contrast, common low-function electric apparatuses are simply configured so as to have a minimum function. Accordingly, such low-function electric apparatuses are made up of only alternate-current component parts. Then, an ion generating device mounted on such an electric apparatus accordingly becomes an ion generating device whose power source is specified as an alternate-current power source.

### Citation List

### [Patent Literature]

[Patent Literature 1] PCT International Application Publication No. WO2015/049933 A1 (Publication Date: April 9, 2015)
[Patent Literature 2] Japanese Patent Application Publication, *Tokukai,* No. 2013-111226 (Publication Date: June 10, 2013)

### Summary of Invention

### Technical Problem

Reduction in size of an electric apparatus requires reduction in size of an ion generating device which is to be mounted on the electric apparatus. In order to decrease the size of the ion generating device, it is necessary to reduce not only the size of a housing constituting the outer shape of the ion generating device but also the size of a substrate etc. which are to be built in the housing. On the substrate, a discharge electrode is mounted. Particularly in a case where both negative and positive ions are to be generated, a discharge electrode for generating positive ions and a discharge electrode for generating negative ions are mounted on the substrate. Reduction in size of the substrate results in a smaller interval between these discharge electrodes.

In an ion generating device whose size reduction is not taken into consideration, an interval between two discharge electrodes is large. Therefore, electronic component parts are provided between the two discharge electrodes, on a substrate. In an ion generating device, high voltage rectifier diodes are provided for respective discharge electrodes. The high voltage rectifier diodes rectify a high alternating-current voltage from a transformer and apply a positive high voltage and a negative high voltage to the two discharge electrodes, respectively. Though these diodes are relatively long, two such diodes can be provided in a linear arrangement in the interval that is large between the discharge electrodes.

However, in a case where an interval between the discharge electrodes is smaller due to reduction in size of the ion generating device, two diodes cannot be provided in a linear arrangement between the discharge electrodes. Accordingly, the diodes need to be provided in a non-linear arrangement or in parallel to each other. In such an arrangement, a voltage application line extending from the transformer is provided near (a tip of) a discharge electrode. Then, when a positive voltage rectified by a diode is applied to one of the discharge electrodes and a negative voltage rectified by another diode is applied to the other one of the discharge electrodes, an electric field weakens because these positive and negative voltages applied to the discharge electrodes are in phase with a voltage of a voltage application path. As a result, ions are not easily generated.

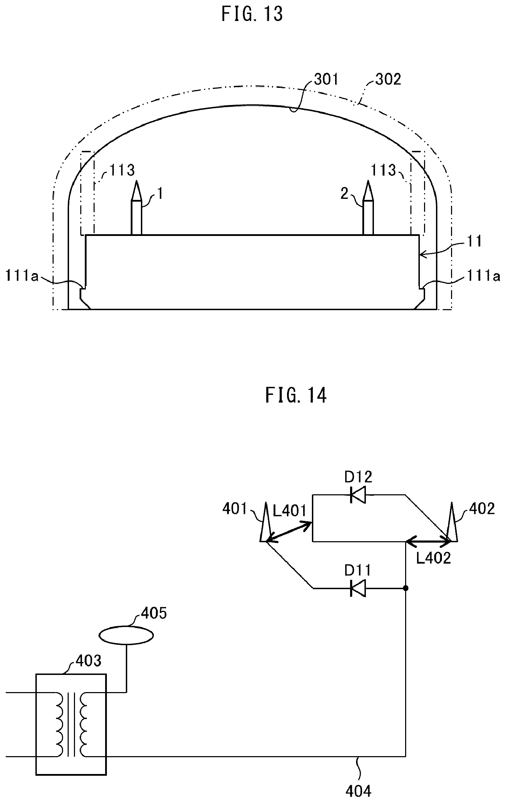

For example, diodes D11 and D12 are provided in parallel to each other in an area between a discharge electrode 401 for generating positive ions and a discharge electrode 402 for generating negative ions, as illustrated in Fig. 14. The diode D11 rectifies an alternating-current voltage from a transformer 403 and applies a resultant positive voltage to the discharge electrode 401. The diode D12 rectifies the alternating-current voltage from the transformer 403 and applies a resultant negative voltage to the discharge electrode 402. One end of a secondary coil of the transformer 403 is connected to an anode of the diode D11 and a cathode of the diode D12 via a wiring pattern 404 which is formed on a substrate. The other end of the secondary coil of the transformer 403 is connected to a counter electrode 405.

In a configuration in which the diodes D11 and D12 are arranged as above, a wiring route between the wiring pattern 404 and the cathode of the diode D12 comes near the discharge electrodes 401 and 402. Accordingly, a smaller interval between the discharge electrodes 401 and 402 leads to a shorter distance L401 between the wiring route and the discharge electrode 401 and a shorter distance L402 between the wiring route and the discharge electrode 402. This makes ion generation more difficult.

Further, in a case where highly-functional electric apparatuses as described above and common electric apparatuses are produced in the same production line, generally, alternate-current (AC) ion generating devices and direct-current (DC) ion generating devices are prepared in the production line, and an AC ion generating device is incorporated into an alternate-current (AC) electric apparatus and a DC ion generating device is incorporated into a direct-current (DC) electric apparatus.

In a case where in order to make component parts common to both AC electronic apparatuses and DC electronic apparatuses, identical housings are used for AC ion generating devices and DC ion generating devices, whether an ion generating device including such a housing is an AC ion generating device or a DC ion generating device cannot be identified from the appearance of the ion generating device. This may cause an AC ion generating device to be incorporated into a DC electric apparatus and/or a DC ion generating device into an AC electric apparatus. In such a case, when an electric apparatus is energized in an operation test, the ion generating device is damaged. On the basis of this result, it is first found that an ion generating device of a wrong power source specification is incorporated in that electric apparatus.

An object of an aspect of the present invention is to provide an ion generating device which can suppress reduction in amount of ions generated and of which size reduction is possible. An object of another aspect of the present invention is to prevent an erroneous connection of a power source of a wrong specification to an ion generating device.

### Solution to Problem

In order to solve the above problems, an ion generating device in accordance with an aspect of the present invention includes: a positive electrode which generates positive ions; a negative electrode which generates negative ions; a transformer which outputs a high alternating-current voltage; a first diode which rectifies the high alternating-current voltage and applies a voltage thus rectified to the positive electrode; and a second diode which rectifies the high alternating-current voltage and applying a voltage thus rectified to the negative electrode, the first diode and the second diode being connected, on a substrate, to an output terminal of the transformer via a conductive body, the conductive body being formed in a region where the conductive body causes neither a decrease in strength of an electric field formed by the positive electrode nor a decrease in strength of an electric field formed by the negative electrode.

In order to solve the above problems, an ion generating device in accordance with another aspect of the present invention includes: a housing including (i) a main body on which electrodes are mounted and (ii) a connector to which a power source for applying voltage to the electrodes is connected, the housing having a power source specification identification section which allows for identification of a power source specification of the ion generating device.

### Advantageous Effects of Invention

An aspect of the present invention advantageously makes it possible to provide an ion generating device which can suppress reduction in amount of ions generated and of which size reduction is possible. Further, another aspect of the present invention advantageously makes it possible to prevent an erroneous connection of a power source of a wrong specification to an ion generating device.

### Brief Description of Drawings

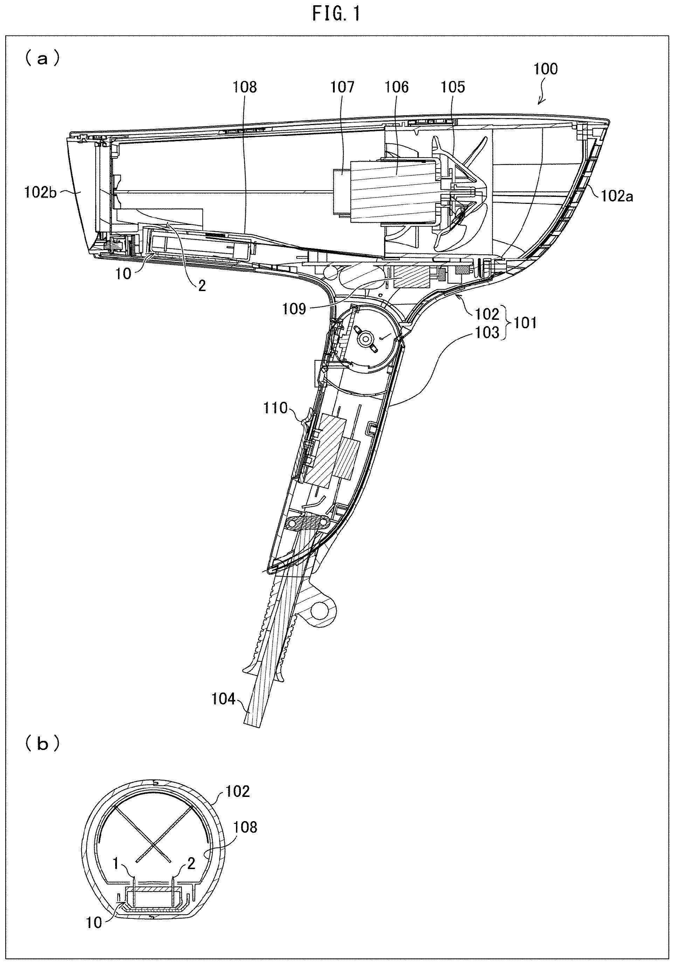

(a) of Fig. 1 is a vertical cross-sectional view illustrating a configuration of a dryer equipped with an ion generating device in accordance with each embodiment of the present invention, and (b) of Fig. 1 is another vertical cross-sectional view illustrating a configuration of the dryer.
Fig. 2 is a perspective view illustrating a configuration of an appearance of an ion generating device in accordance with any one of Embodiments 1 to 3 of the present invention.
Fig. 3 is a circuit diagram illustrating a configuration of an electric system of the ion generating device in accordance with Embodiment 1.
Fig. 4 is a view illustrating an arrangement example of diodes in the ion generating device illustrated in Fig. 3.
(a) of Fig. 5 is a view illustrating another arrangement example of diodes in the ion generating device illustrated in Fig. 3, and (b) of Fig. 5 is a view illustrating still another arrangement example of diodes in the ion generating device illustrated in Fig. 3.
Fig. 6 is a vertical cross-sectional view illustrating a configuration of an ion generating device in accordance with a Variation of Embodiment 1.
(a) of Fig. 7 is a plan view illustrating a housing of the ion generating device in accordance with Embodiment 2 of the present invention, and (b) of Fig. 7 is a plan view illustrating another housing of the ion generating device in accordance with Embodiment 2.
(a) of Fig. 8 is a side view illustrating a housing of the ion generating device in accordance with Embodiment 2 of the present invention, and (b) of Fig. 8 is a side view illustrating another housing of the ion generating device in accordance with Embodiment 2.
(a) of Fig. 9 is a plan view illustrating a housing of the ion generating device in accordance with Embodiment 2 of the present invention, and (b) of Fig. 9 is a plan view illustrating another housing of the ion generating device in accordance with Embodiment 2.
(a) to (c) of Fig. 10 is a view illustrating respective shapes of connectors of the ion generating device in accordance with Embodiment 2 of the present invention.

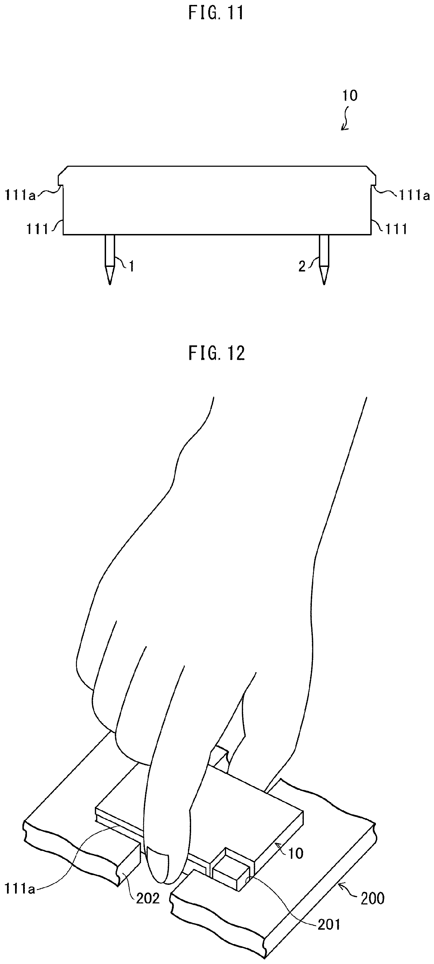

Fig. 11 is an elevational view illustrating a configuration of the ion generating device in accordance with Embodiment 3 of the present invention.
Fig. 12 is a perspective view illustrating a state in which the ion generating device illustrated in Fig. 11 is being pulled out from a tray.
Fig. 13 is a view illustrating a state in which the ion generating device illustrated in Fig. 11 is provided in an air path of an electric apparatus.
Fig. 14 is a view illustrating an arrangement of diodes in a conventional ion generating device.

### Description of Embodiments

### [Dryer]

With reference to Fig. 1, the following will discuss a dryer incorporating an ion generating device 10 (10A) in accordance with any of Embodiments 1 to 3 of the present invention. (a) of Fig. 1 is a vertical cross-sectional view illustrating a configuration of a dryer equipped with an ion generating device in accordance with each embodiment of the present invention. (b) of Fig. 1 is another vertical cross-sectional view illustrating a configuration of the dryer.

As illustrated in (a) and (b) of Fig. 1, a dryer 100 includes a casing 101. The casing 101 includes a main body cylinder portion 102 and a handle portion 103. From the handle portion 103, a power supply cable 104 is drawn.

The main body cylinder portion 102 is provided, at one end thereof, an inlet 102a for drawing in air and, at the other end thereof, an outlet 102b for blowing out the air. The main body cylinder portion 102 is provided with an air blowing fan 105, a motor 106, a heater unit 107, and an ion generating device 10 in an area from the inlet 102a to the outlet 102b inside the main body cylinder portion 102. The motor 106 causes the air blowing fan 105 to rotate. When the air blowing fan 105 rotates, an air flow from the inlet 102a to the outlet 102b is produced and heat generated by the heater unit 107 is sent as hot air toward the outlet 102b.

Further, the main body cylinder portion 102 is provided therein a heat-insulating sleeve 108 from the motor 106 to the outlet 102b. The heat-insulating sleeve 108 forms a path for wind generated by the air blowing fan 105, in other words, an air path.

In the vicinity of the outlet 102b, the ion generating device 10 is provided between the main body cylinder portion 102 and the heat-insulating sleeve 108. The ion generating device 10 includes a discharge electrode 1 (positive electrode) for generating positive ions and a discharge electrode 2 (negative electrode) for generating negative ions. The discharge electrodes 1 and 2 are exposed to the outside of the ion generating device 10 such that tips of the discharge electrodes 1 and 2 reach inside the heat-insulating sleeve 108 as illustrated in (b) of Fig. 1. The ion generating device 10 generates ions inside the heat-insulating sleeve 108 and spreads the ions in a flow of air passing inside the heat-insulating sleeve 108.

Further, the main body cylinder portion 102 is provided therein with a circuit substrate 109 in the vicinity of a portion where the main body cylinder portion 102 and the handle portion 103 are joined to each other. In a case where the dryer 100 is a common dryer, a motor driving circuit is mounted on the circuit substrate 109. The motor driving circuit drives the motor 106. The motor driving circuit controls a rate of rotation of the motor 106 so as to change an air volume sent by the air blowing fan 105 in accordance with the strength of hot wind which strength is changed by operating a switch 110 (described later). The power supply cable 104 supplies an alternate current power. The alternating-current power is directly supplied to the motor 106, the heater unit 107 and the ion generating device 10 from the power supply cable 104. In contrast, in a case where the dryer 100 is a highly-functional dryer, a power source of the dryer 100 is specified as a direct-current power source. Therefore, on the circuit substrate 109, a motor driving circuit, an AC/DC converter, a control circuit, etc. are mounted. The AC/DC converter converts, into a direct-current power, an alternating-current power supplied via the power supply cable 104, and then supplies the direct-current power to the control circuit and to the ion generating device 10. The control circuit includes a CPU etc., and controls respective operations of the motor 106, the heater unit 107, and the ion generating device 10.

The handle portion 103 is provided with the switch 110. The switch 110 is provided so as to switch on/off the power source and to change the strength of the hot wind.

Note that an electric apparatus on which the ion generating device 10 is mounted is not limited to the dryer 100, and can be another electric apparatus.

### [Embodiment 1]

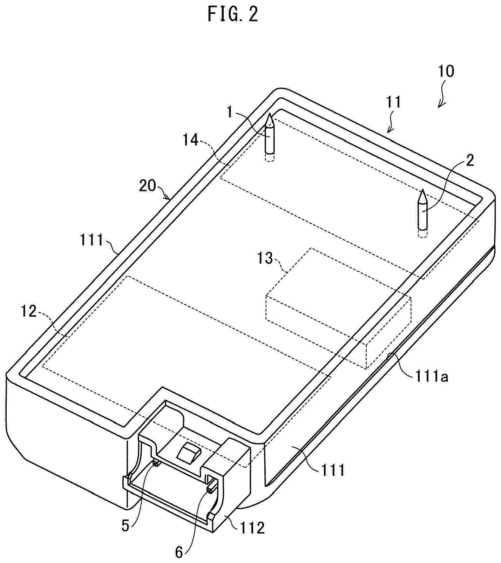

The following description will discuss an embodiment of the present invention with reference to Figs. 2 to 6.

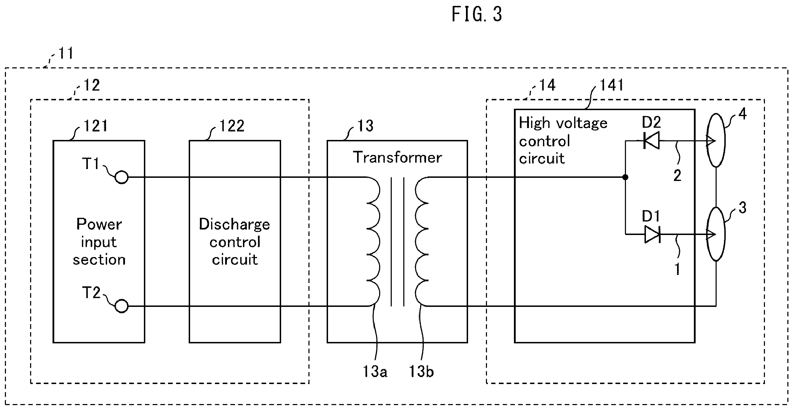

Fig. 2 is a perspective view illustrating a configuration of an appearance of an ion generating device 10 in accordance with Embodiment 1. Fig. 3 is a circuit diagram illustrating a configuration of an electric system of the ion generating device 10.

As illustrated in Fig. 2, the ion generating device 10 includes a housing 11 which is made of a resin material. The housing 11 includes a main body portion 20 which in whole forms a rectangular box shape, and a connector 112. The main body portion 20 has two side surfaces 111 which are opposed to each other and which are along the longest side of three sides of the main body portion 20. These side surfaces 111 are each provided with a step 111a along a long-side direction of the side surfaces 111. The connector 112 is provided on one of side surfaces along the second shortest side of the three sides of the housing 11. The connector 112 is joined to a connector (not illustrated) which is connected to a power supply cable. The connector 112 is provided therein with pins 5 and 6.

The housing 11 contains a discharge control circuit substrate 12, a transformer 13, and a high voltage circuit substrate 14. As illustrated in Fig. 3, in the ion generating device 10, a power input section 121 and a discharge control circuit 122 are mounted on the discharge control circuit substrate 12, and a high voltage control circuit 141, discharge electrodes 1 and 2 and induction electrodes 3 and 4 are mounted on the high voltage circuit substrate 14. As illustrated in Fig. 2, the discharge electrodes 1 and 2 are needle-like electrodes whose tips are sharp-pointed. The discharge electrodes 1 and 2 are provided so as to be spaced apart from each other by a predetermined distance.

Note that the induction electrodes 3 and 4 may not be mounted on the high voltage circuit substrate 14, and may be mounted on another substrate.

In a case where the power source of the ion generating device 10 is specified as a direct-current power source, the power input section 121 is a portion at which a direct-current voltage from the connector 112 is inputted to the discharge control circuit substrate 12. The power input section 121 includes terminals T1 and T2. The terminal T1 is connected to the pin 5 of the connector 112 and the terminal T2 is connected to the pin 6 of the connector 112.

The discharge control circuit 122 is a circuit which drives the transformer 13 by (i) converting, into an alternating-current voltage at a predetermined frequency, the direct-current voltage having been thus inputted from the connector 112 and (ii) then applying the alternating-current voltage to a primary coil 13a of the transformer 13. The discharge control circuit 122 is connected to the primary coil 13a (low voltage side) of the transformer 13.

Note that in a case where the power source of the ion generating device 10 is specified as an alternating-current power source, an alternating-current voltage can be inputted to the connector 112 (power input section 121). In such a case, the discharge control circuit 122 has, for example, a current-limiting resistor for limiting an input current, a rectifying circuit, and a switching circuit.

The high voltage control circuit 141 includes a diode D1 (first diode) and a diode 2 (second diode). The high voltage control circuit 141 is a circuit which, after rectifying a high alternating-current voltage which is to be subsequently outputted from one of terminals (output terminals) of a secondary coil 13b (high voltage side) of the transformer 13, applies a positive voltage to the discharge electrode 1 and a negative voltage to the discharge electrode 2. The diodes D1 and D2 are high voltage rectifier diodes and have the same length.

To one terminal of the high voltage control circuit 141, an anode of the diode D1 and a cathode of the diode D2 are connected. Meanwhile, a cathode of the diode D1 is connected to the discharge electrode 1 and an anode of the diode D2 is connected to the discharge electrode 2. Both of the induction electrodes 3 and 4 are connected to the other one of the output terminals of the transformer 13. The induction electrode 3 is provided in the vicinity of the discharge electrode 1, and the induction electrode 4 is provided in the vicinity of the discharge electrode 2.

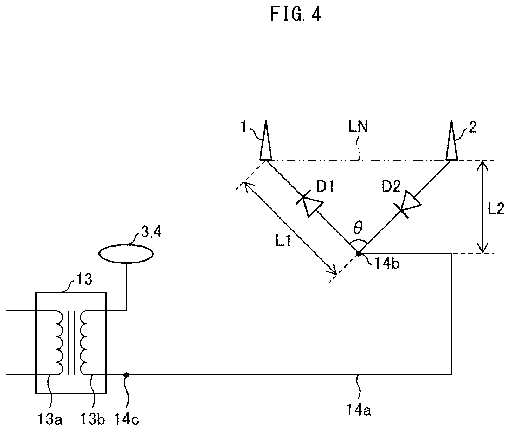

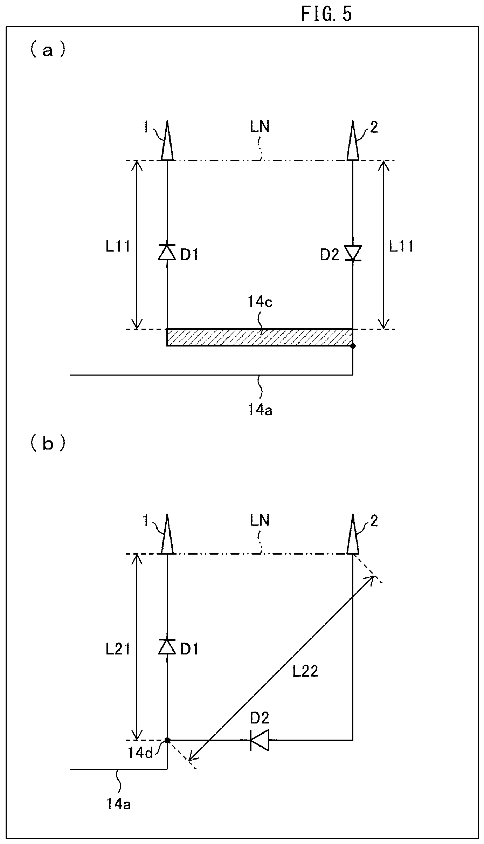

Next, the following will discuss an arrangement of the diodes D1 and D2 on the high voltage circuit substrate 14. Fig. 4 is a view illustrating an arrangement example of the diodes D1 and D2. (a) of Fig. 5 is a view illustrating another arrangement example of the diodes D1 and D2, and (b) of Fig. 5 is a view illustrating still another arrangement example of the diodes D1 and D2.

On the high voltage circuit substrate 14, a wiring pattern 14a and soldering patterns 14b and 14c are formed as illustrated in Fig. 4. The wiring pattern 14a is provided so as to connect the one of the terminals of the secondary coil 13b of the transformer 13 to both of the anode of the diode D1 and the cathode of the diode D2. The soldering pattern 14b is a punctate part at which an end of an anode lead of the diode D1 and an end of a cathode lead of the diode D2 are connected to one end of the wiring pattern 14a. The soldering pattern 14c is a punctate part at which the one of terminals (leads) of the secondary coil 13b of the transformer 13 is soldered to the other end of the wiring pattern 14a.

The diodes D1 and D2 are arranged to incline with respect to a straight line LN connecting between the discharge electrodes 1 and 2. In this arrangement, the diodes D1 and D2 are provided so as to be connected to the soldering pattern 14b such that the diodes D1 and D2 form the shape of the letter V. Further, an angle θ made between the diodes D1 and D2 is most preferably 90° at the soldering pattern 14b.

The discharge electrodes 1 and 2 each form an electric field which extends around from a tip of the discharge electrode 1 or 2. Since these electric fields of opposite polarities affect each other between the discharge electrodes 1 and 2, an electric field between the discharge electrodes 1 and 2 is intensified. On the other hand, in a case where another electric field (in this case, electric field caused by a potential of the secondary coil 13b of the transformer 13) different from the electric fields formed by the discharge electrodes 1 and 2 exist in an area between the discharge electrodes 1 and 2, the electric field by the discharge electrode 1 or the electric field by the discharge electrode 2 is momentarily weakened.

In contrast, in the arrangement example illustrated in Fig. 4, the electric field between the discharge electrodes 1 and 2 is not weakened. This is because there is no conductive body whose potential is the same as that of the secondary coil 13b of the transformer 13, in a region where the conductive body might weaken the electric field between the discharge electrodes 1 and 2. Further, the diodes D1 and D2 are inclined with respect to the straight line LN as in the arrangement example illustrated in Fig. 4. In this arrangement, the soldering pattern 14b is a conductive body closest to the discharge electrode 1, which conductive body has the same potential as the secondary coil 13b of the transformer 13 and which conductive body is connected to the diode D1. Therefore, it can be ensured that a distance L1 between the discharge electrode 1 and the soldering pattern 14b is substantially the same as the length of the diode D1. In addition, in the arrangement, the wiring pattern 14a is a conductive body closest to the discharge electrode 2, which conductive body has the same potential as the secondary coil 13b of the transformer 13 and which conductive body is connected to the diode D2. Therefore, a distance L2 between the discharge electrode 2 and the wiring pattern 14a can be ensured. This makes it possible to prevent influence of a conductive body having the same potential as the secondary coil 13b of the transformer 13 to the electric field between the discharge electrodes 1 and 2.

As a result, the electric field formed by the discharge electrodes 1 and 2 is prevented from weakening. This makes it possible to suppress reduction in amount of ions generated by the discharge electrodes 1 and 2. Further, it is also possible to shorten the distance between the straight line LN and the soldering pattern 14b. This makes it possible to prevent an increase in width of the high voltage circuit substrate 14.

Alternatively, the diodes D1 and D2 may be arranged as illustrated in (a) or (b) of Fig. 5.

As illustrated in (a) of Fig. 5, the diodes D1 and D2 are arranged to be perpendicular to a straight line LN, and also to be parallel to each other. An end of an anode lead of the diode D1 and an end of a cathode lead of the diode D2 are connected to respective ends of a linear soldering pattern 14c. The soldering pattern 14c is a part which is formed on the high voltage circuit substrate 14 so as to be parallel to a straight line LN and at which the anode lead of the diode D1 and the cathode lead of the diode D2 are soldered to one end of a wiring pattern 14a. In this arrangement, the diodes D1 and D2 are connected to the wiring pattern 14c so as to form the shape of the letter U with the wiring pattern 14c. Meanwhile, there is no conductive body whose potential is the same as that of the secondary coil 13b of the transformer 13, between the discharge electrodes 1 and 2.

In this arrangement example, as with the arrangement example illustrated in Fig. 4, there is no conductive body whose potential is the same as that of the secondary coil 13b of the transformer 13, between the discharge electrodes 1 and 2. Therefore, an electric field between the discharge electrodes 1 and 2 is not weakened in this arrangement example illustrated in (a) of Fig. 5. Further, in this arrangement example, the diodes D1 and D2 are arranged to be perpendicular to the straight line LN. Therefore, the soldering pattern 14c is a conductive body closest to the discharge electrodes 1 and 2, which conductive body has the same potential as the secondary coil 13b of the transformer 13 and which conductive body is connected to the diode D1. Accordingly, it can be ensured that a distance L11 between the discharge electrode 1 and the soldering pattern 14c is substantially the same as the length of the diode D1 and a distance L11 between the discharge electrode 2 and the soldering pattern 14c is substantially the same as the length of the diode D2. This makes it possible to prevent influence of a conductive body having the same potential as the secondary coil 13b of the transformer 13 to the electric field between the discharge electrodes 1 and 2 in this arrangement example more than in the arrangement example illustrated in Fig. 4. As a result, the electric field formed by the discharge electrodes 1 and 2 is prevented from weakening. This makes it possible to suppress reduction in amount of ions generated by the discharge electrodes 1 and 2.

Note that the high voltage circuit substrate 14 in the arrangement example illustrated in (a) of Fig. 5 has a larger width than that in the arrangement example illustrated in Fig. 4, since the diodes D1 and D2 are arranged to be perpendicular to the straight line LN in this arrangement example.

In an arrangement illustrated in (b) of Fig. 5, the diode D1 is arranged to be perpendicular to a straight line LN and the diode D2 is arranged to be parallel to the straight line LN. An end of an anode lead of the diode D1 and an end of a cathode lead of the diode D2 are connected to a soldering pattern 14d. Further, an end of an anode lead of the diode D2 is connected to the discharge electrode 2 via a wiring pattern (different from a wiring pattern 14a described below) which is formed on a high voltage circuit substrate 14. The soldering pattern 14d is a punctate part which is formed on the high voltage circuit substrate 14 and at which the anode lead of the diode D1 and the cathode lead of the diode D2 are soldered to one end of the wiring pattern 14a. In this arrangement, the diodes D1 and D2 are provided so as to be connected to the soldering pattern 14d such that the diodes D1 and D2 form the shape of the letter L. Meanwhile, there is no conductive body whose potential is the same as that of the secondary coil 13b of the transformer 13, between the discharge electrodes 1 and 2.

In this arrangement example, the diode D1 is arranged to be perpendicular to the straight line LN. Therefore, the soldering pattern 14d is a conductive body closest to the discharge electrode 1, which conductive body has the same potential as the secondary coil 13b of the transformer 13 and which conductive body is connected to the diode D1. Therefore, it can be ensured that a distance L21 between the discharge electrode 1 and the soldering pattern 14d is substantially the same as the length of the diode D1. Further, on the assumption that the straight line LN is a first side of a right triangle, a line whose length is equal to the total length of the diode D1 is a second side of the right triangle, and the first and second sides together make a right angle, it can be ensured that a distance L22 between the discharge electrode 2 and the soldering pattern 14d is substantially the same as the length of a third side of that right triangle. The distance L22 is longer than the distance L21.

In this arrangement example, as with the arrangement example illustrated in Fig. 4, there is no conductive body whose potential is the same as that of the secondary coil 13b of the transformer 13, between the discharge electrodes 1 and 2. Therefore, an electric field formed by the discharge electrodes 1 and 2 is not weakened in this arrangement example illustrated in (b) of Fig. 5. Further, in this arrangement example, as with the arrangement example illustrated in Fig. (b) of Fig. 5, it is possible to have a longer distance between each of the discharge electrodes 1 and 2 and a conductive body whose potential is the same as that of the secondary coil 13b of the transformer 13. This makes it possible to prevent influence of such a conductive body to the electric field. As a result, the electric field formed by the discharge electrodes 1 and 2 is prevented from weakening. This makes it possible to suppress reduction in amount of ions generated by the discharge electrodes 1 and 2.

Note that the high voltage circuit substrate 14 in the above arrangement example illustrated in (b) of Fig. 5 also has a larger width than that in the arrangement example illustrated in Fig. 4, since the diode D1 is arranged to be perpendicular to the straight line LN in this arrangement example.

Here, the following will discuss a distance between the wiring pattern 14a and the soldering pattern 14b which are illustrated in Fig. 4 and a distance between the wiring pattern 14a and the discharge electrodes 1 and 2.

In the ion generating device 10, the distance between the wiring pattern 14a and the soldering pattern 14b in the arrangement example illustrated in Fig. 4 is longer in a portion where the discharge electrodes 1 and 2 are provided, as compared to a distance between a wiring pattern and a soldering pattern in a conventional arrangement example illustrated in Fig. 14. Further, in the ion generating device 10, the distance between the wiring pattern 14a and each of the tips of the discharge electrodes 1 and 2 in the arrangement example illustrated in Fig. 4 is longer in a portion where the discharge electrodes 1 and 2 are provided, as compared to a distance between each of tips of the discharge electrodes 401 and 402 and a wiring pattern 14a and a soldering pattern in the conventional arrangement example illustrated in Fig. 14.

### <Variation>

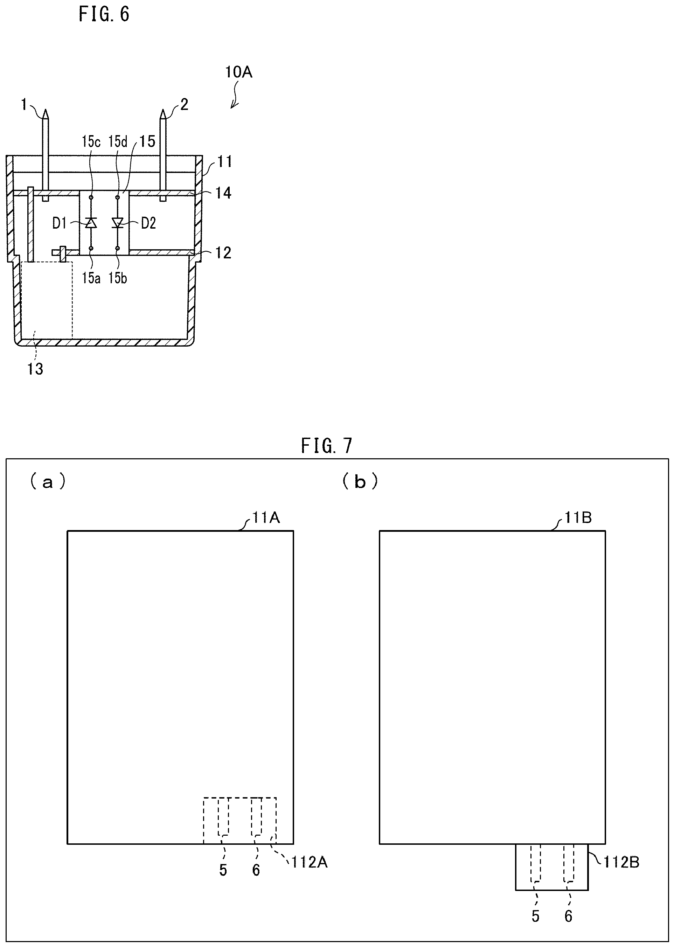

The following description will discuss a Variation of Embodiment 1. Fig. 6 is a vertical cross-sectional view illustrating a configuration of an ion generating device in accordance with the present Variation.

As illustrated in Fig. 6, an ion generating device 10A in accordance with the present Embodiment is larger in thickness (depth) in a direction in which discharge electrodes 1 and 2 extend, as compared to the ion generating device 10 described above. Further, in the ion generating device 10A, a discharge control circuit substrate 12 is provided so as to be spaced apart from a high voltage circuit substrate 14 by a predetermined distance, in the direction in which the discharge electrodes 1 and 2 extend. Furthermore, the discharge control circuit substrate 12 and the high voltage circuit substrate 14 are connected with an intermediate substrate 15. The intermediate substrate 15 is provided so as to be perpendicular to the high voltage circuit substrate 14.

On the intermediate substrate 15, diodes D1 and D2 are mounted. Further, on the intermediate substrate 15, soldering patterns 15a to 15d are formed. The soldering pattern 15a is a punctate part at which an end of an anode lead of the diode D1 is soldered to one end of the above-described wiring pattern 14a. The soldering pattern 15a is provided on a discharge control circuit substrate 12 side. The soldering pattern 15b is a punctate part at which an end of a cathode lead of the diode D2 is soldered to one end of the wiring pattern 14a. The soldering pattern 15b is provided on the discharge control circuit substrate 12 side. The soldering pattern 15c is a punctate part at which an end of a cathode lead of the diode D1 is soldered to a wiring pattern (not illustrated) on the high voltage circuit substrate 14 which wiring pattern is drawn from the discharge electrode 1. The soldering pattern 15c is provided on a high voltage circuit substrate 14 side. The soldering pattern 15d is a punctate part at which an end of an anode lead of the diode D2 is soldered to a wiring pattern (not illustrated) on the high voltage circuit substrate 14 which wiring pattern is drawn from the discharge electrode 2. The soldering pattern 15d is provided on the high voltage circuit substrate 14 side.

In the ion generating device 10 configured as above, there is no conductive body whose potential is the same as that of a secondary coil of a transformer 13, between the discharge electrodes 1 and 2. Further, in the ion generating device 10, the diodes D1 and D2 are mounted on the intermediate substrate 15 and the soldering patterns 15a and 15b are provided below the discharge electrodes 1 and 2 in Fig. 6. As compared to the arrangement examples illustrated in Figs. 4 and 5, the soldering patterns 15a and 15b, which have the same potential as the secondary coil of the transformer 13, are farther from the discharge electrodes 1 and 2 (particularly from tips of the discharge electrodes 1 and 2) in the ion generating device 10A.

This makes it possible to further suppress reduction in amount of ions generated by the discharge electrodes 1 and 2, as compared to the arrangement examples illustrated in Figs. 4 and 5. In addition, since the diodes D1 and D2 are not mounted on the high voltage circuit substrate 14, it is possible to decrease the width of the high voltage circuit substrate 14.

Note that though the intermediate substrate 15 is provided so as to be perpendicular to the high voltage circuit substrate 14 (surface on which the discharge electrodes 1 and 2 are mounted) in the present Variation, the intermediate substrate 15 can be arranged so as to incline with respect to the high voltage circuit substrate 14.

### [Embodiment 2]

Next, the following will discuss Embodiment 2 of the present invention, with reference to Figs. 7 to 10. Note that, for convenience of explanation, identical reference numerals are given to members which have respective functions identical with those described in Embodiment 1, and descriptions of the respective members are omitted.

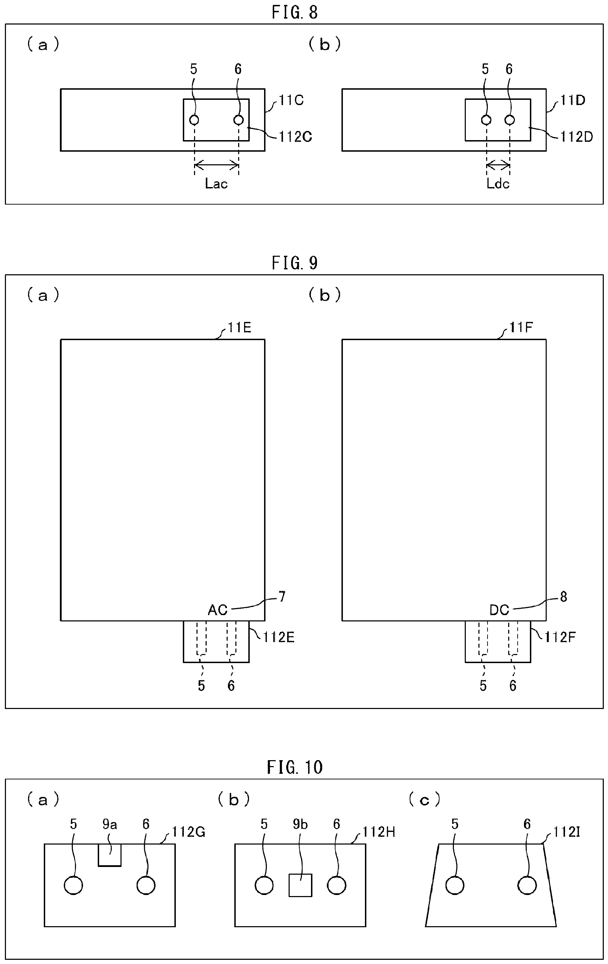

(a) of Fig. 7 is a plan view illustrating a housing 11A of an ion generating device 10 in accordance with Embodiment 2. (b) of Fig. 7 is a plan view illustrating another housing 11B of the ion generating device 10. (a) of Fig. 8 is a side view illustrating a housing 11C of the ion generating device 10 in accordance with Embodiment 2. (b) of Fig. 8 is a side view illustrating another housing 11D of the ion generating device 10. (a) of Fig. 9 is a plan view illustrating a housing 11E of the ion generating device 10 in accordance with Embodiment 2. (b) of Fig. 9 is a plan view illustrating another housing 11F of the ion generating device 10. (a) to (c) of Fig. 10 is a view illustrating respective shapes of connectors 112G to 1121 of the ion generating device 10 in accordance with Embodiment 2.

The housing 11A is a housing which is included in the ion generating device 10 that is an AC device, and has a connector 112A as illustrated in (a) of Fig. 7. The connector 112A is formed so as to be depressed inward in the housing 11A. The connector 112A is provided with pins 5 and 6. A connector (hereinafter, referred to as an AC connector) (not illustrated) of an AC power supply cable wired from the circuit substrate 109 of the above-described dryer 100 is shaped so as to fit in the connector 112A.

In contrast, the housing 11B is a housing which is included in the ion generating device 10 that is a DC device, and has a connector 112B as illustrated in (b) of Fig. 7. Further, the housing 11B is shaped so as to have the same shape and the same size as the housing 11A. The connector 112B is formed so as to protrude outside the housing 11B and have a cavity inside the connector 112B. In this cavity, the pins 5 and 6 are provided. A connector (hereinafter, referred to as an DC connector) (not illustrated) of a DC power supply cable wired from the circuit substrate 109 of the above-described dryer 100 is shaped so as to fit to the connector 112B outside the connector 112B.

In this way, with regard to the housing 11A that is an AC housing and the housing 11B that is a DC housing, the shape of the connector 112A is different from the shape of the connector 112B and accordingly, the shape of the AC connector of the power supply cable is different from the shape of the DC connector of the power supply cable. Therefore, the shape of the connector 112A and the shape of the connector 112 make it possible to distinguish between respective power source specifications corresponding to the connectors 112A and 112B. As a result, the DC power supply cable cannot be connected to the connector 112A and the AC power supply cable cannot be connected to the connector 112B. This makes it possible to easily identify the power source specification of the ion generating device 10. Consequently, it is possible to prevent an erroneous connection of a power source to the ion generating device 10.

Further, the housing 11C is a housing which is included in the ion generating device 10 that is an AC device, and has a connector 112C as illustrated in (a) of Fig. 8. The connector 112C is provided with pins 5 and 6 such that a distance between the pins 5 and 6 is a distance Lac. Correspondingly, two holes (not illustrated) into which the pins 5 and 6 are to be fit, respectively, are provided in the AC connector such that a distance between the two holes is also the distance Lac.

In contrast, the housing 11D is a housing which is included in the ion generating device 10 that is a DC device, and has a connector 112D. Further, the housing 11D is shaped so as to have the same shape and the same size as the housing 11C. The connector 112D is provided with pins 5 and 6 such that a distance between the pins 5 and 6 is a distance Ldc. Correspondingly, two holes (not illustrated) into which the pins 5 and 6 are to be fit, respectively, are provided in the DC connector such that a distance between the two holes is also the distance Ldc.

The distance Lac between the pins 5 and 6 provided in the connector 112C is longer than the distance Ldc between the pins 5 and 6 provided in the connector 112D. Since an alternating-current power supply voltage is higher than a direct-current power supply voltage, the distance Lac is arranged to be longer than the distance Ldc so that a sufficient withstand voltage will be ensured. As a result, the DC power supply cable cannot be connected to the connector 112C and the AC power supply cable cannot be connected to the connector 112D. Consequently, it is possible to prevent an erroneous connection of a power source to the ion generating device 10.

In examples illustrated in Figs. 7 and 8, in molding the housings 11A to 11D, a mold for molding a main body of a housing is shared by the housings 11A to 11D regardless of whether the specification is an AC specification or a DC specification, and different molds are used for molding the connectors 112A to 112D depending on whether the specification of a connector to be molded is the AC specification or the DC specification. This makes it possible to reduce mold cost. Therefore, it is possible to reduce cost of the ion generating device 10.

Further, the housing 11E is a housing which is included in the ion generating device 10 that is an AC device, and has a connector 112E as illustrated in (a) of Fig. 9. The connector 112E is formed so as to protrude outside the housing 11E and have a cavity inside the connector 112E. In this cavity, pins 5 and 6 are provided. The housing 11E is provided with a power source specification indicating section 7 (power source specification identification section, display) in the vicinity of the connector 112E. In the power source specification indicating section 7, the letters "AC", which indicate that the power source specification is the AC specification, are printed.

In contrast, the housing 11F is a housing which is included in the ion generating device 10 that is a DC device, and has a connector 112F as illustrated in (b) of Fig. 9. The connector 112F is formed so as to protrude outside the housing 11E and have a cavity inside the connector 112F. In this cavity, pins 5 and 6 are provided. The connector 112F is arranged such that the shape of the connector 112F is the same as the shape of the connector 112E and that a distance between the pins 5 and 6 in the connector 112F is also the same as that in the connector 112E. Therefore, the DC connector to be connected to the connector 112F also has the same shape as the AC connector to be connected to the connector 112E. However, the housing 11F is provided with a power source specification indicating section 8 (power source specification identification section, display) in the vicinity of the connector 112F. In the power source specification indicating section 8, the letters "DC", which indicate that the power source specification is the DC specification, are printed.

In this way, the housing 11E is provided with the power source specification indicating section 7 which indicates that the power source specification is the AC specification, whereas the housing 11F is provided with the power source specification indicating section 8 which indicates that the power source specification is the DC specification. This allows a worker in an assembly line to identify whether an ion generating device 10 is an AC device or a DC device, by checking an indication of the power source specification indicating section 7 or 8, when the worker installs the ion generating device 10 in a dryer 100. As a result, it is possible to prevent an erroneous connection of a power source to the ion generating device 10. Further, the housings 11E and 11F have the same shape and the same size (the connectors 112E and 112F of the housings 11E and 11F also have the same shape and the same size). Therefore, it is possible to use one mold for molding both the housings 11E and 11F. This makes it possible to further reduce cost required for molds and consequently to reduce the cost of the ion generating device 10.

Note that in the power source specification indicating sections 7 and 8, the indication is not limited to letters and can be a sign, a pattern, or the like which allows for distinction between the AC specification and the DC specification. Further, in the power source specification indicating sections 7 and 8, letters or the like can be engraved other than printed.

Further, a housing 11 of the ion generating device 10 that is an AC device can have a connector 112G as illustrated in (a) of Fig. 10, whereas the housing 11 of the ion generating device 10 that is a DC device can have a connector 112H as illustrated in (b) of Fig. 10. The connector 112G has a rectangular shape when viewed from a tip side of the pins 5 and 6 and a projection 9a is formed on a wall surface along a long side of this rectangular shape. In an AC connector which is to be fit in the connector 112G, a depressed portion is formed. The projection 9a is to be fit in this depressed portion of the AC connector. In contrast, the connector 112H has a rectangular shape when viewed from a tip side of the pins 5 and 6 and a projection 9b is formed between the pins 5 and 6. In a DC connector which is to be fit into the connector 112H, a depressed portion is formed. The projection 9b is to be fit into this depressed portion of the DC connector.

Because positions of the projections 9a and 9b are different from each other as described above, the DC power supply cable cannot be connected to the connector 112G and the AC power supply cable cannot be connected to the connector 112H. As a result, it is possible to prevent an erroneous connection of a power source to the ion generating device 10.

Note that the projections 9a and 9b can be arranged to have the same or different shapes and the same or different sizes. Alternatively, projections can be provided at identical positions so as to have different shapes depending on respective power source specifications.

It is also possible to arrange such that the housing 11 of the ion generating device 10 that is an AC device has the connector 112H whereas the housing 11 of the ion generating device 10 that is a DC device has the connector 112G.

Alternatively, the housing 11 of the ion generating device 10 that is an AC device can have a connector 1121 as illustrated in (c) of Fig. 10. The connector 1121 has a trapezoidal shape when viewed from a tip side of the pins 5 and 6. Then, a corresponding AC connector which is to be fit in the connector 112G also has a trapezoidal shape.

In contrast, the housing 11 of the ion generating device 10 that is a DC device can have a connector which has a rectangular shape when viewed from a tip side of the pins 5 and 6 (rectangular connector), as illustrated in (a) of Fig. 8. Then, a corresponding DC connector which is to be fit in the rectangular connector also has a rectangular shape.

Since the connector 1121 has a structure whose outer shape is different from that of the above-described rectangular connector, the DC power supply cable cannot be connected to the connector 1121 and the AC power supply cable cannot be connected to the rectangular connector. As a result, it is possible to prevent an erroneous connection of a power source to the ion generating device 10.

Note that it is also possible to arrange such that the housing 11 of the ion generating device 10 that is an AC device has the rectangular connector whereas the housing 11 of the ion generating device 10 that is a DC device has the connector 112I.

Further, in order to distinguish between the AC specification and the DC specification, one of the above-described differences in connector shape, in distance between pins, and in display of letters or the like can be used or some or all of the above-described differences can be used in combination. By using such differences in combination, it is possible to more reliably prevent an erroneous connection of a power source.

### [Embodiment 3]

Next, the following will discuss Embodiment 3 of the present invention, with reference to Figs. 2, and 11 to 13. Note that, for convenience of explanation, identical reference numerals are given to members which have respective functions identical with those described in Embodiment 1 or 2, and descriptions of the respective members are omitted.

Fig. 11 is an elevational view illustrating a configuration of an ion generating device 10 in accordance with Embodiment 3. Fig. 11 illustrates a side surface of the ion generating device 10, which side surface is on an opposite side of a side surface where a connector 112 illustrated in Fig. 2 is provided. Fig. 12 is a perspective view illustrating a state in which the ion generating device 10 illustrated in Fig. 11 is being pulled out from a tray. Fig. 13 is a view illustrating a state in which the ion generating device 10 illustrated in Fig. 11 is provided in an air path 301 of an electric apparatus.

As illustrated in Figs. 2 and 11, each side surface 111, which is along the longest side of three sides of the housing 11, is provided with a step 111a which is formed so as to extend along a long-side direction of the side surface 111. The width of the step 111a is, for example, 0.2 mm, and can be in a range of some hundred micrometers.

A general ion generating device often has a housing whose structure is simple and whose surface is flat. Though a relatively large ion generating device can be held by an entire hand, an ion generating device having a smaller housing needs to be pinched with fingertips when handled. Particularly, when the housing has a flat surface, the ion generating device is slippery and difficult to pinch.

Further, an ion generating device which utilizes electric discharge has discharge electrodes which are designed to protrude from the housing. The discharge electrodes are often sharp-pointed and easily damaged. In light of this, in a conventional ion generating device, for example, a housing is provided with a discharge electrode protecting section at positions lateral to the discharge electrodes, as disclosed in Patent Literature 1. The discharge electrode protecting section is intended to protect the discharge electrodes and to prevent a discharge electrode(s) from being touched.

However, since such a discharge electrode protecting section is provided at lateral side portions of the housing, the discharge electrode protecting section hinders size reduction of the ion generating device. Further, the discharge electrode protecting section not only makes the structure of the housing more complex but also hinders electric discharge.

In contrast, the ion generating device 10 in accordance with Embodiment 3 is not provided with the discharge electrode protecting section. Further, the ion generating device 10 has the above-described steps 111a. Since fingers are easily hooked on the steps 111a, the steps 111a prevent the ion generating device 10 pinched with fingers from being dropped.

For example, when the ion generating device 10 is carried, a plurality of ion generating devices 10 is contained in a tray 200 as (partially) illustrated in Fig. 12. In the tray 200, a plurality of depressed sections 201 is provided in the tray 200 (only one depressed section 201 is illustrated in Fig. 12). In each of the depressed sections 201, the ion generating device 10 is fit so as not to easily slip out of the depressed section 201. When the ion generating device 10 is pulled out from the tray 200, fingers are put in a groove 202 of the tray 200 and the ion generating device 10 is pinched on both sides with the fingers. At this time, the ion generating device 10 can be firmly held with the fingers hooked on the steps 111a. Accordingly, the fingers do not slip on the ion generating device 10 against holding force of the depressed section 201 of the tray 200, and the ion generating device 10 can be easily pulled out from the tray 200. Therefore, it is possible to easily prevent the ion generating device 10 from being slipped from the fingers and dropped, when the ion generating device 10 is pulled out from the tray 200.

Further, since the ion generating device 10 does not have a discharge electrode protecting section, the ion generating device 10 has a reduced size. This makes it possible to easily incorporate the ion generating device 10 in a compact electric apparatus. For example, a hair iron or the like has a narrower air path than the above-described dryer 100. As illustrated in Fig. 13, since the ion generating device 10 can be put in a narrow air path 301, the ion generating device 10 can be provided in a hair iron or the like.

In contrast, in a case where discharge electrode protecting sections 113 are provided at respective lateral side portions of the housing 11, the discharge electrode protecting sections 113 cannot be contained within the air path 301. Accordingly, in order to contain the discharge electrode protecting sections 113 in the air path 301, an air path 302 which is wider than the air path 301 is required. This disadvantageously causes an electric apparatus to have a larger size.

As described above, since the ion generating device 10 in accordance with Embodiment 3 has the step 111a, it is possible not only to easily prevent the ion generating device 10 from being dropped during handling of the ion generating device 10 but also to easily reduce the size of the ion generating device 10.

### [Recap]

An ion generating device in accordance with Aspect 1 of the present invention includes: a positive electrode (discharge electrode 1) which generates positive ions; a negative electrode (discharge electrode 2) which generates negative ions; a transformer 13 which outputs a high alternating-current voltage; a first diode (diode D1) which rectifies the high alternating-current voltage and applies a voltage thus rectified to the positive electrode; and a second diode (diode D2) which rectifies the high alternating-current voltage and applying a voltage thus rectified to the negative electrode, the first diode and the second diode being connected, on a substrate (high voltage circuit substrate 14), to an output terminal of the transformer 13 via a conductive body (wiring pattern 14a and soldering patterns 14b to 14d), the conductive body being formed in a region where the conductive body causes neither a decrease in strength of an electric field formed by the positive electrode nor a decrease in strength of an electric field formed by the negative electrode.

In the above configuration, the conductive body whose potential equals to that of the output terminal of the transformer is formed in a region where the conductive body does not weaken the electric fields. Accordingly, it is possible to prevent the electric fields from weakening due to influence of the conductive body. This makes it possible to suppress reduction in amount of ions generated by the positive electrode and the negative electrode. Further, the first diode and the second diode are appropriately arranged in accordance with the position of the conductive body. This makes it possible to reduce the size of the ion generating device.

An ion generating device in accordance with Aspect 2 of the present invention can be arranged such that: in Aspect 1, the first diode and the second diode are provided so as to incline with respect to a straight line connecting between the positive electrode and the negative electrode.

In the above configuration, the conductive body is placed far from the positive electrode and the negative electrode. This can reduce influence of the conductive body to the electric fields and also can reduce the width of the substrate on which the first diode and the second diode are mounted.

An ion generating device in accordance with Aspect 3 of the present invention can be arranged such that: in Aspect 1, at least one of the first diode and the second diode is provided so as to be perpendicular to a straight line connecting between the positive electrode and the negative electrode.

In the above configuration, the conductive body is placed farther from the positive electrode and the negative electrode, as compared to Aspect 2. This make is possible to further reduce influence of the conductive body to the electric fields.

An ion generating device in accordance with Aspect 4 of the present invention can be arranged such that: in Aspect 1, the substrate is provided so as to be perpendicular to or incline with respect to a surface on which the positive electrode and the negative electrode are mounted.

In the above configuration, the conductive body can be placed farther from the positive electrode and the negative electrode. This makes it possible to further reduce influence of the conductive body to the electric fields.

An ion generating device in accordance with Aspect 5 of the present invention can be arranged to further include, in any of Aspects 1 to 4, a housing 11 on which the positive electrode, the negative electrode, the transformer 13, the first diode, and the second diode are mounted, the housing 11 being provided with a step 111a on each of two side surfaces 111 of the housing 11 which side surfaces 111 are opposed to each other.

With the above configuration, even in a case where the size of the housing is reduced in accordance with size reduction of the ion generating device, fingers can be easily hooked on the housing. Accordingly, even when the ion generating device is pinched with fingers, the ion generating device is not easily dropped. Further, since the ion generating device is not easily dropped, it is possible to avoid damage to the positive electrode and the negative electrode due to dropping of the ion generating device. This eliminates the need for a structure for protection of the positive electrode and the negative electrode in the ion generating device. This makes it possible to easily reduce the size of the ion generating device.

An ion generating device in accordance with Aspect 6 of the present invention includes: a housing 11 including (i) a main body 20 on which electrodes (discharge electrodes 1 and 2) are mounted and (ii) a connector 112 to which a power source for applying voltage to the electrodes is connected, the housing 11 having a power source specification identification section (connectors 112A to 1121, power source specification indicating sections 7 and 8, and projections 9a and 9b) which allows for identification of a power source specification of the ion generating device.

With the above configuration, even in a case where the housing is formed so as to have an identical shape and an identical size regardless of a power source specification of the ion generating device, the power source specification of the ion generating device can be identified by the power source specification identification section. This makes it possible to prevent an erroneous connection of a power source to the ion generating device.

An ion generating device in accordance with Aspect 7 of the present invention can be arranged such that: in Aspect 6, the power source specification identification section is the connector 112 (connectors 112A and 112B) which is formed into a shape associated with the power source specification.

With the above configuration, the power source specification of the ion generating device can be identified by difference in the shape of the connector. Due to the difference, the connector of the ion generating device cannot be connected with a power supply cable whose power source specification is different from that of the ion generating device. Accordingly, an erroneous connection of a power source to the ion generating device can be prevented.

An ion generating device in accordance with Aspect 8 of the present invention can be arranged such that: in Aspect 6, the power source specification identification section is an interval between two pins 5 and 6 which are provided in the connector 112 so as to be spaced apart from each other by a distance associated with the power source specification.

With the above configuration, the power source specification of the ion generating device can be identified by difference in the interval between the pins of the connector. Due to the difference, the connector of the ion generating device cannot be connected with a power supply cable whose power source specification is different from that of the ion generating device. Accordingly, an erroneous connection of a power source to the ion generating device can be prevented.

An ion generating device in accordance with Aspect 9 of the present invention can be arranged such that: in Aspect 6, the power source specification identification section is a display associated with the power source specification.

With the above configuration, the power source specification of the ion generating device can be identified by the difference in the display associated with the power source specification. This makes it possible to visually check the power source specification of the ion generating device. This makes it possible to prevent an erroneous connection of a power source to the ion generating device.

An ion generating device in accordance with Aspect 10 of the present invention can be arranged such that: in Aspect 6, the power source specification identification section is a projection 9a, 9b, which has a shape associated with the power source specification, which is provided at a position associated with the power source specification, or which has a shape associated with the power source specification and also is provided at a position associated with the power source specification.

With the above configuration, the power source specification of the ion generating device can be identified by difference in at least one of the shape of the projection and the position of the projection. Due to the difference, the connector of the ion generating device cannot be connected with a power supply cable whose power source specification is different from that of the ion generating device. Accordingly, an erroneous connection of a power source to the ion generating device can be prevented.

An ion generating device in accordance with Aspect 11 of the present invention can be arranged such that: in any one of Aspects 6 to 9, the housing 11 is provided with a step 111a on each of two side surfaces 111 of the housing 11 which side surfaces 111 are opposed to each other.

With the above configuration, even in a case where the size of the housing is reduced in accordance with size reduction of the ion generating device, fingers can be easily hooked on the housing. Accordingly, even when the ion generating device is pinched with fingers, the ion generating device is not easily dropped. Further, since the ion generating device is not easily dropped, it is possible to avoid damage to the positive electrode and the negative electrode due to dropping of the ion generating device. This eliminates the need for a structure for protection of the positive electrode and the negative electrode in the ion generating device. This makes it possible to easily reduce the size of the ion generating device.

### [Supplemental Remarks]

The present invention is not limited to the embodiments, but can be altered by a skilled person in the art within the scope of the claims. The present invention also encompasses, in its technical scope, any embodiment derived by combining technical means disclosed in differing embodiments. Further, it is possible to form a new technical feature by combining the technical means disclosed in the respective embodiments.

### Reference Signs List

1 discharge electrode (positive electrode)
2 discharge electrode (negative electrode)
7, 8 power source specification indicating section (power source specification identification section, display)
9a, 9b projection (power source specification identification section)
10, 10A ion generating device
11, 11A to 11F housing
13 transformer
14 high voltage circuit substrate (substrate)
14a wiring pattern (conductive body)
14b to 14d soldering pattern (conductive body)
15 intermediate substrate (substrate)
20 main body
111 side surface
111a step
112A to 1121 connector (power source specification identification section)
D1 diode (first diode)
D2 diode (second diode)
LN straight line

## Claims

1. An ion generating device comprising:
a positive electrode which generates positive ions;
a negative electrode which generates negative ions;
a transformer which outputs a high alternating-current voltage;
a first diode which rectifies the high alternating-current voltage and applies a voltage thus rectified to the positive electrode; and
a second diode which rectifies the high alternating-current voltage and applying a voltage thus rectified to the negative electrode,
the first diode and the second diode being connected, on a substrate, to an output terminal of the transformer via a conductive body,
the conductive body being formed in a region where the conductive body causes neither a decrease in strength of an electric field formed by the positive electrode nor a decrease in strength of an electric field formed by the negative electrode.

2. The ion generating device as set forth in claim 1, wherein:
the first diode and the second diode are provided so as to incline with respect to a straight line connecting between the positive electrode and the negative electrode.

3. The ion generating device as set forth in claim 1, wherein:
at least one of the first diode and the second diode is provided so as to be perpendicular to a straight line connecting between the positive electrode and the negative electrode.

4. The ion generating device as set forth in claim 1, wherein:
the substrate is provided so as to be perpendicular to or incline with respect to a surface on which the positive electrode and the negative electrode are mounted.

5. An ion generating device as set forth in any one of claims 1 to 4, further comprising:
a housing on which the positive electrode, the negative electrode, the transformer, the first diode, and the second diode are mounted,
the housing being provided with a step on each of two side surfaces of the housing which side surfaces are opposed to each other.

6. An ion generating device comprising:
a housing including (i) a main body on which electrodes are mounted and (ii) a connector to which a power source for applying voltage to the electrodes is connected,
the housing having a power source specification identification section which allows for identification of a power source specification of the ion generating device.

7. The ion generating device as set forth in claim 6, wherein:
the power source specification identification section is the connector which is formed into a shape associated with the power source specification.

8. The ion generating device as set forth in claim 6, wherein:
the power source specification identification section is an interval between two pins which are provided in the connector so as to be spaced apart from each other by a distance associated with the power source specification.

9. The ion generating device as set forth in claim 6, wherein:
the power source specification identification section is a display associated with the power source specification.

10. The ion generating device as set forth in claim 6, wherein:
the power source specification identification section is a projection which has a shape associated with the power source specification, which is provided at a position associated with the power source specification, or which has a shape associated with the power source specification and also is provided at a position associated with the power source specification.

11. The ion generating device as set forth in any one of claims 6 to 10, wherein:
the housing is provided with a step on each of two side surfaces of the housing which side surfaces are opposed to each other.
